# EUROPEAN PATENT APPLICATION

(11) **EP 1 457 197 A1**
(43) Date of publication of application: **15.09.2004**
(21) Application number: 03005588.3
(22) Date of filing: 12.03.2003
(51) Int. Cl.: A61K 7/13

(54) **Composition for dyeing human hair**

(71) Applicant: KPSS-Kao Professional Salon Services GmbH, 64297 Darmstadt (DE)
(72) Inventor: Wilz, Rüdiger, 64319 Pfungstadt (DE)

(57) **Abstract**

The present invention relates to an oxidative hair dyeing composition comprising 2,6-hydroxyethylamino toluene and/or its salts. Oxidative hair dyeing composition of the invention comprises further developing and coupling substances.

## Description

The present invention concerns a composition for the dyeing of human hair on the basis of an oxidation dyestuff precursor system reacting with peroxide which provides long-lasting, intensive colors either used as such, or which can be used to obtain further shades in combination with additional developing and/or coupling agents and which does not damage the hair even upon repeated application within short intervals.

The developing substances still most frequently used in hair dyeing compositions are 1,4-diaminobenzene (p-phenylenediamine) and 1-methyl-2,5-diaminobenzene (p-toluylenediamine). Although incorporation of these substances largely fulfills the user's color wishes, there are still shades that cannot be completely achieved by the use thereof.

Proposals have also been made to close this gap by the use of alternative developing substances. To a limited degree this is possible with the use of tetraaminopyrimidine or 2-(2,5-diaminophenyl)ethanol (see. EP-B 400, 330); however, it is then necessary to accept reduced color intensity in other shades.

A further satisfactory solution of this problem is disclosed in EP-A 615 743, with the use of 2-(2'-hydroxyethyl amino)-5-aminotoluene or the water-soluble salts thereof, and the triaminohydroxy pyrimidines, in particular 2,5,6-triamino-4-hydroxy pyrimidine, 2,4,5-triamino-6-hydroxy pyrimidine, 4,5,6-triamino-2-hydroxy pyrimidine or the salts thereof, in particular the sulfates, known from EP-B 467 026 as developing substances in hair dyeing compositions. However, even these proposals fail to fulfill all wishes for hair colors.

As a component of oxidation dyestuffs it has also been proposed to use 3,4-diamino-5-hydroxypyrazol. However, to date it has not been possible to prepare strong colorations by use of these substances.

The invention starts from the task of counteracting this deficiency and providing an oxidation dyestuff composition which provides intensive, glossy colorations.

This task is solved when such a hair dyeing composition comprises an oxidation dyestuff 2,6-dihydroxyethylamino toluene as developer. In addition to that it is also possible to incorporate further developing and coupling substances.

After oxidation with peroxide, use of these compositions on the basis of a customary carrier provides very expressive, intensive, long-lasting hair colorations, which can be varied to achieve further shades by the addition of the respective further developing and coupling substances.

It is also possible to incorporate further developing substances known **per se**.
Preferred as such are 1,4 diaminobenzene and substituted p-phenylenediamines, in particular 2,5-diamino-toluene, 2-n-propyl or 2-ethyl-p-phenylenediamine, 2,6-di-methyl-p-phenylenediamine, 2-(2,5-diaminophenyl) ethanol; 1-amino-4-bis-(2'-hydroxy-ethyl)aminobenzene, 2-(2-hydroxyethyl amino)-5-aminotoluene, 4,4'-di-aminodiphenylamine, 4-aminodiphenylamine, 2-amino-5-N,N-diethyl amino-toluene, 4-amino-N-ethyl-N-isopropyl aniline, 2-chloro-p-phenylenediamine, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene, 1-amino-4-β-hydroxypropyl aminobenzene 2-aminophenol, 4-aminophenols and the derivatives thereof, tetraamino pyrimidines, triamino hydroxy pyrimidines, diamino dihydroxy pyrimidines, 5-amino salicylic acid and/or 1,2,4-triaminobenzenes or the water-soluble salts thereof.

The composition according to the invention comprises coupling substance, which can be selected from resorcinol, 2-methyl resorcinol, 4-chlororesorcinol, 2-amino-4-chlorophenol; 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hydroxyethylamino)benzene, 1-amino-3-[bis(2'-hydroxy-ethyl) amino]benzene, α-naphtho), 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol and/or 1-methoxy-2-amino-4-(2'-hydroxyethyl amino)benzene or the water-soluble salts thereof

The total concentration of the developing substances customarily ranges between about 0.05 % and 5 %, preferably 0.1 % and 4 %, in particular 0.25 % to 0.5 % and 2.5 % to 3 % by weight, calculated to the total hair dyeing composition (excluding the oxidation agent), whereby these figures are always related to the proportion of free base. The preferred weight proportion of the developing substances to the additional developing and coupling substances ranges between about 1 : 8 to 8 : 1, preferably about 1 : 5 to 5 : 1, in particular 1 : 2 to 2 : 1.

In the hair dyeing compositions according to the invention, the coupling substance(s) as reaction partners of the developing substance(s) are present in approximately the same molecular proportions as the developing substances, i.e. in amounts from 0.05 % to 5.0 %, preferably 0.1 % to 4 %, in particular 0.5 % to 3 % by weight, calculated to the total composition (excluding the oxidizing agent), whereby these figures are always related to the proportion of free base.

If desired, the compositions according to the invention can also contain so-called shading agents for precise adjustment of the desired shade, in particular direct-acting dyestuffs.

Such shading agents are, for example, nitro dyestuffs such as 2-amino-4,6-dinitrophenol, 2-amino-4-nitrophenol, 2-amino-6-chloro-4-nitrophenol, etc., preferably in amounts from about 0.05 % to 2.5 %, in particular 0.1 % to 1 % by weight of the dyestuff composition (excluding the oxidizing agent).

The hair dyeing compositions according to the invention can comprise the basic substances and additives customarily found in such compositions, conditioning agents, etc., known as state of the art and described, for example, in the monography of K. Schrader, "Grundlagen und Rezepturen der Kosmetika", 2nd Ed. (Hüthig Buch Verlag, Heidelberg, 1989), pp. 782 to 815. They can be prepared as solutions, creams, gels or also in the form of aerosol products; suitable carrier material compositions are known as state of the art.

For application, the oxidation dyestuff precursor is mixed with an oxidizing agent. The preferred oxidizing agent is hydrogen peroxide, for example in a concentration of 2 % to 6 % by weight.
However, the use of other peroxides such as urea peroxide and melamine peroxide is also possible.

The pH-value of the ready-to-use hair dyeing composition, i.e. after mixing with peroxide, can be in a slightly acidic range, i.e. from 5.5 to 6.9, as well as in the neutral or alkaline range, i.e. between pH 7.1 and 10.

In the following, various Examples are used to illustrate the invention.

| **Carrier** | |
|---|---|
| Stearyl alcohol | 8.0 (% by wt.) |
| Coco fatty acid monoethanolamide | 4.5 |
| 1,2-Propanediol mono/distearate | 1.3 |
| Coco fatty alcohol polyglycolether | 4.0 |
| Sodium lauryl sulfate | 1.0 |
| Oleic acid | 2.0 |
| 1.2-Propanediol | 1.5 |
| Na-EDTA | 0.5 |
| Sodium sulfite | 1.0 |
| Protein hydrolyzate | 0.5 |
| Ascorbic acid | 0.2 |
| Perfume | 0.4 |
| Ammonia, 25% | 1.0 |
| Ammonium chloride | 0.5 |
| Panthenol | 0.8 |
| Water | ad 100.00 |

The oxidation dyestuff combinations according to the invention were incorporated into this carrier, whereby the water content was reduced accordingly.

The colorations were carried out on wool patches and strands of bleached human hair by application of a 1:1 mixture of a dyestuff precursor and a 6 % hydrogen peroxide solution (pH-value of the mixture: 9.8) with twenty minutes processing at room temperature, subsequent rinsing and drying.

The following colorations were achieved:

### Example 1:

| | |
|---|---|
| 22.5 mMol | 2,6-dihydroxyethylamino toluene |
| 22.5 mMol | 2,5-diaminotoluene sulphate |
| | |

**Coloration:** Intensive purple violet

### Example 2:

| | |
|---|---|
| 22.5 mMol | 2,6-dihydroxyethylamino toluene |
| 22.5 mMol | 4-amino-m-cresol |
| | |

**Coloration:** Intensive purple

### Example 3:

| | |
|---|---|
| 22.5 mMol | 2,6-dihydroxyethylamino toluene |
| 22.5 mMol | (2-hydroxyethyl)-2,5-diaminobenzol sulphate |
| | |

**Coloration:** Intensive red violet

### Example 4:

| | |
|---|---|
| 22.5 mMol | 2,6-dihydroxyethylamino toluene |
| 22.5 mMol | 4-hydroxy-2,5,6-triaminopyrimidine |
| | |

**Coloration:** Transparent light red

### Example 5:

| | |
|---|---|
| 22.5 mMol | 2,6-dihydroxyethylamino toluene |
| 22.5 mMol | 3,chlor-4-aminophenol hydrochloride |
| | |

**Coloration:** Deep magenta

## Claims

1. Oxidative hair dyeing composition **characterised in that** it comprises as a developing substance 2,6-hydroxyethylamino toluene and/or its salts.

2. Oxidative hair dyeing composition according to claim 1 comprises further developing and/or coupling substances.

3. Oxidative hair dyeing composition according to claim 1 comprises developing substances at a total concentration of 0.05 % and 5 %, preferably 0.1 % and 4 %, in particular 0.25 % to 3 % by weight, calculated to the total hair dyeing composition (excluding the oxidation agent), whereby these figures are always related to the proportion of free base.

4. Oxidative hair dyeing composition according to claim 1 comprises coupling substances at a total concentration of 0.05 % and 5 %, preferably 0.1 % and 4 %, in particular 0.25 % to 3 % by weight, calculated to the total hair dyeing composition (excluding the oxidation agent), whereby these figures are always related to the proportion of free base.
